# EUROPEAN PATENT SPECIFICATION

(11) **EP 2 460 514 B1**
(45) Date of publication and mention of the grant of the patent: **28.08.2013**
(21) Application number: 11191683.9
(22) Date of filing: 02.12.2011
(51) Int. Cl.: A61K 9/06, A61K 31/00, A61K 47/00, A61K 31/726

(54) **Topical pharmaceutical gel compositions of flurbiprofen, glucosamine and chondroitin**
Topische pharmazeutische Gelzusammensetzungen aus Fluriprofen, Glucosamin und Chondroitin
Compositions de gel pharmaceutique topique de flurbiprofène, glucosamine et chondroïtine

(30) Priority: 03.12.2010 TR 201010074
(43) Date of publication of application: 06.06.2012
(73) Proprietor: Sanovel Ilaç Sanayi Ve Ticaret Anonim Sirketi, 34460 Sariyer/Istanbul (TR)
(72) Inventor: CIFTER, Ümit, 34398 Istanbul (TR); TÜRKYILMAZ, Ali, 34398 Istanbul (TR); AKALIN, M Nur Pehlivan, 34398 Istanbul (TR); ZENGINER, M Sibel, 34398 Istanbul (TR); ÖNER, Levent, 34398 Istanbul (TR)
(74) Representative: Sevinç, Erkan

(56) References cited:
- WO-A1-03/099013
- WO-A2-2010/013279
- US-A1- 2008 227 747

## Description

### Field of Invention

The present invention relates to topical pharmaceutical gel compositions comprising flurbiprofen or pharmaceutically acceptable salts thereof with glucosamine or salts thereof and chondroitin sulfate. More specifically, the invention relates to topical pharmaceutical gel compositions comprising flurbiprofen or pharmaceutically acceptable salts thereof, glucosamine or salts thereof, chondroitin sulfate and dimethyl sulfoxide. Furthermore, the invention relates to process for preparing the said topical pharmaceutical gel compositions and its use for the treatment of osteoarthritis, pain and inflammatory symptoms associated with joint and cartilage disorders.

### Background of Invention

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains. Its chemical structure is illustrated with Formula I given below.

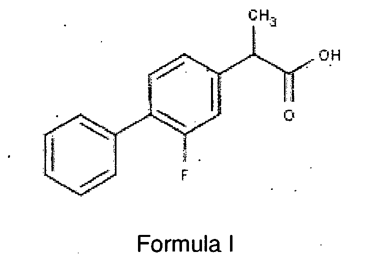

Flurbiprofen is mostly administrated orally. One disadvantage of the oral administration of flurbiprofen comprising compositions is that the patient is likely to experience unpleasant side effects, including gastrointestinal irritation.

The use of flurbiprofen in treating local pains and inflammations may cause a problem especially for those who have gastrointestinal system disorders. It is possible to develop various locally-administrable topical forms of flurbiprofen, in order to avoid the systemic side-effects thereof. The skin absorption rate of the relevant product to be used in topical applications, however, is quite significant. Enhancing the absorption rate both provides ease of application and increases the molecule's efficiency.

Glucosamine is an amino sugar and aprominent precursor in the biochemical synthesis of glycosylated proteins and lipids. Glucosamine is part of the structure of the polysaccharides chitosan and chitin and it is naturally present in the shells of shellfish, animal bones and bone marrow. It is also present in some fungi and can be also synthetically derived. The preferred salts of glucosamine include N-acetyl-glucosamine, glucosamine hydrochloride and glucosamine sulfate and mixtures thereof. Glucosamine is mostly administrated orally for the treatment of osteoarthritis.

Chondoritin sulfate is a sulfated glycosaminoglycan (GAG) composed of a chain of alternating sugars. Chondroitin sulfate is an important structural component of cartilage and provides much of its resistance to compression. Chondroitin sulfate is mostly administrated orally for the treatment of osteoarthritis.

It is known that, glucosamine and chondroitin are apparently poor candidates for transdermal absorbtion, and they are not preferred in topical compositions. Therefore, the requirement is obvious to increase the rate of absorption of them in topical compositions.

Accordingly, in acute or chronic disorders, there arises the need of enhancing the absorption rate at the site of administration. For instance, during which local pains associated with injuries in sportive events are to be urgently alleviated, it becomes necessary to apply local anesthesia to the relevant site.

In prior art, there are also several patents which disclose NSAIDs in combination with glucosamine and chondroitin mostly in oral pharmaceutical dosage forms but none of them disclose the topical pharmaceutical gel compositions or none of them selected particularly flurbiprofen to combine with glucosamine and chondroitin in a gel composition because of their poor transdermal absorption properties.

US 2008/0227747 A1, disclose a therapeutic composition and methods for the treatment and prevention of a degenerative joint disorder and/or a cardiovascular disease comprising polycosanols, glucosamine and chondroitin adapted for oral administration. But it is silent about the use of glucosamine and chondroitin in combination with flurbiprofen in topical compositions particularly in acute joint disorders.

PCT patent application WO 03/099013, disclose a dietary supplement comprising natural ingredients such as glucosamine and derivatives in combination with acetaminophen or acetylsalicylic acid further comprising chondroitin. This application is not directed to a topical gel composition which comprise flurbiprofen in combination with glucosamine and chondroitin with special excipients such as dimethyl sulfoxide to enhance their percutaneous penetration rate. It is objected to provide oral pharmaceutical dosage forms such as dietary regimen and formulation having improved gastrointestinal absorbtion.

Accordingly, there exists need for a percutaneous delivery system for the drug flurbiprofen, glucosamine and chondroitin which optimizes the delivery of them through the skin.

It is therefore an object of the present invention to provide a topical pharmaceutical gel composition of flurbiprofen or pharmaceutically acceptanle salts thereof, glucosamine or salts thereof and chondroitin sulfate which is more effective for purposes of percutaneous delivery of them through the skin.

### Summary of the Invention

The present invention relates to an easily applicable flurbiprofen or pharmaceutically acceptable salts thereof, glucosamine or salts thereof and chondroitin sulfate topical pharmaceutical gel composition, which overcomes the above described problems in prior art and have additive advantages over them.

Accordingly, the main object of the present invention is to increase the rate of percutaneous penetration, thereby shortening the time period in which the active agents exert their effect.

The rate of percutaneous penetration of said combination is enhanced with the dimethyl sulfoxide.

Another object of the present invention is to obtain a stable topical pharmaceutical gel composition of flurbiprofen, glucosamine and chondroitin during the shelf-life and to exhibit high safety when applied to skin.

Another object of the present invention is to obtain the viscosity in a stable level to enhance the penetration of the topical gel composition by the help of lecithin and carboxyl methyl cellulose. They enhance the penetration properties of the composition when used with dimethyl sulfoxide.

A further object of the present invention is to obtain a formulation with local anesthetic effect, with the menthol used in said combination stimulating the receptors by which the cold sensation is perceived.

In a preferred embodiment according to the present invention, said novelty is realized with a topical pharmaceutical gel composition comprising 0.50 to 30.0% by weight of flurbiprofen or a pharmaceutically acceptable salt thereof, 0.50 to 20.0% by weight of glucosamine or salts thereof, 0.10 to 20.0% by weight of chondroitin sulfate, 0.10 to 30.0% by weight of dimethyl sulfoxide.

According to a preferred embodiment of the present invention the amount of dimethyl sulfoxide is preferably from 0.50 to 25.0% by weight of the total composition, more preferably it is from 1.0 to 20.0% by weight of the total composition, most preferably it is from 5.0 to 15.0% by weight of the total composition.

According to a preferred embodiment, the glucosamine salt is glucosamine sulfate.

According to a preferred embodiment, the topical pharmaceutical gel composition of the invention further comprises lecithin in the amount of from 0.10 to 20.0% by weight of the total composition.

According to a preferred embodiment, the topical pharmaceutical gel composition of the invention further comprises sodium carboxyl methyl cellulose in the amount of from 0.05 to 10.0% by weight of the total composition.

According to a preferred embodiment, the weight ratio of lecithin to sodium carboxyl methyl cellulose is from 25:1 to 1:25 by weight, preferably it is from 10:1 to 1:10 by weight of the total composition.

According to a preferred embodiment of the present invention, the topical pharmaceutical gel composition further comprises menthol, wherein the amount of menthol is from 0.10 to 15.0% by weight of the total composition, preferably it is from 0.50 to 10.0% by weight of the total composition.

According to a preferred embodiment of the present invention, the topical pharmaceutical gel composition, further comprises surface active agents, viscosity enhancers, chelating agents, aromatic agents, preservatives, dissolving solvents, antioxidants and their mixtures. According to a preferred embodiment, the present invention comprise disodium edetate as the chelating enhancer, wherein the amount is from 0.005 to 5.0% by weight of the total composition, preferably it is from 0.05 to 2.0% by weight of the total composition.

In a further preferred embodiment of the present invention, said topical pharmaceutical gel composition comprise the following;

| | | |
|---|---|---|
| a. | flurbiprofen or pharmaceutically acceptable salts thereof | 0.50 to 30.0% by weight |
| b. | glucosamine sulfate | 0.50 to 20.0% by weight |
| c. | chondroitin sulfate | 0.10 to 20.0% by weight |
| d. | dimethyl sulfoxide | 0.10 to 30.0% by weight |
| e. | lecithin | 0.10 to 20.0% by weight |
| f. | fenouil oil | 0.01 to 15.0% by weight |
| g. | eucalyptus oil | 0.01 to 15.0% by weight |
| h. | carnation oil | 0.01 to 15.0% by weight |
| i. | ginger oil | 0.01 to 15.0% by weight |
| j. | lavender oil | 0.01 to 15.0% by weight |
| k. | sweet orange oil | 0.10 to 15.0% by weight |
| l. | menthol | 0.10 to 15.0% by weight |
| m. | methyl paraben | 0.01 to 2.0% by weight |
| n. | propyl paraben | 0.01 to 2.0% by weight |
| o. | disodium edetate | 0.005 to 5.0% by weight |
| p. | propylene glycol | 1.0 to 30.0% by weight |
| q. | polyethylene glycol | 1.0 to 50.0% by weight |
| r. | sodium carboxyl methyl cellulose | 0.05 to 10.0% by weight |
| s. | purified water | 1.0 to 50.0% by weight |
| t. | ethyl alcohol | 1.0 to 75.0% by weight |

Another aspect of the present invention provides a method for preparing the topical pharmaceutical gel composition according to the present invention, this method comprising the steps of;
a. mixing flurbiprofen, methyl paraben, propyl paraben and menthol in ethyl alcohol until they dissolve. Then adding fenouil oil, eucalyptus oil, carnation oil, ginger oil, lavender oil, sweet orange oil, disodium edetate and lecithin and mixing them 10 more minutes to obtain the mixture 1.
b. adding water to another container and mixing chondroitin sulfate and glucosamine sulfate in this container until they dissolve. Then adding sodium carboxyl methyl cellulose, propylene glycol, polyethylene glycol and dimethyl sulfoxide and mixing them 30 more minutes to obtain mixture 2.
c. adding the second mixture into the first mixture and stirring 10 more minutes.

According to another preferred embodiment of the present invention, the topical pharmaceutical gel composition is used in the treatment of osteoarthritis, pain and inflammatory symptoms associated with joint and cartilage disorders.

Further advantages and embodiments of the present invention will become apparent from the following description

### Detailed Description of Invention

According to the present invention, a novel formulation with anti-inflammatory and analgesic activities is obtained surprisingly, which is rapidly absorbed and gives local anesthetic effect.

The flurbiprofen useful in accordance with this invention includes the pharmaceutically acceptable salts and esters of flurbiprofen, and further includes the conventionally used racemic mixture which comprises the S- and R- enantiomers of flurbiprofen. The topical pharmaceutical gel compositions of the invention comprise from 0.50 to 30.0% flurbiprofen, preferably from 1.0 to 20.0%, more preferably from 2.0 to 10.0%.

The topical pharmaceutical gel compositions of the invention comprise from 0.10 to 20.0% chondroitin sulfate, preferably from 0.5 to 15.0%, more preferably from 0.75 to 10.0% by weight of the total composition and comprise from 0.50 to 20.0% glucosamine or salts thereof, preferably from 1.0 to 10.0% by weight of the total composition.

The topical pharmaceutical gel compositions of the invention comprise from 0.10 to 30.0% dimethyl sulfoxide, preferably from 0.50 to 25.0%, more preferably from 1.0 to 20.0%, most preferably about 5.0 to 15.0% by weight of the total composition. Dimethyl sulfoxide helps the composition of the present invention to improve and enhance the penetrating and spreading properties through the skin. It also helps to carry out other components easily and without damaging the membranes into biological system. Accordingly, dimethyl sulfoxide is used as a topical analgesic, a vehicle for topical application of pharmaceuticals, as an anti-inflammatory and an antioxidant. These properties, surprisingly is found to have synergistic effect over flurbiprofen and chondroitin/glucosamine percutaneous penetration properties through the skin.

The topical pharmaceutical gel compositions of the invention comprise from 0.10 to 20.0% lecithin, preferably from 1.0 to 15.0% by weight, more preferably from 2.0 to 10.0% by weight of the total composition, most preferably from 5.0 to 10.0% by weight of the total composition.

The topical pharmaceutical gel compositions of the invention comprise from 0.05 to 10.0% sodium carboxyl methyl cellulose, preferably from 0.50 to 5.0% by weight of the total composition, more preferably from 0.50 to 2.0% by weight of the total composition.

Surprisingly it is found that when the weight ratio of lecithin to sodium carboxyl methyl cellulose is from 25:1 to 1:25 by weight, preferably from 10:1 to 1:10; it enhance the penetration properties of the formulation in combination with dimethyl sulfoxide. Accordingly, lecithin and sodium carboxyl methyl cellulose help to obtain the viscosity in a stable level to enhance the penetration of the topical composition and their stabilizer and emulsifier property is also help them to show this effect easily.

Furthermore, the topical pharmaceutical compositions of the invention comprise menthol from 0.10 to 15.0%, preferably from 0.50 to 10.0%, more preferably about 1.0 to 5.0% by weight of the total composition. Menthol used in the formulation gives anesthetic effect at the side of administration as a result of stimulating the receptors by which cold sensation is perceived.

Menthol when used with chondroitin sulfate and glucosamine sulftate, provides significant relief of pain associated with mild to moderate muscle strain in adult patients. Also menthol helps to mask the bad odour of the other excipients to obtain a good patient compliance when applying to skin.

The pharmaceutical compositions according to the present invention may also comprise one or more pharmaceutically acceptable excipients. Such proper pharmaceutically acceptable excipients comprise, but are not limited to surface active agents, viscosity enhancers, chelating agents, aromatic agents, preservatives, dissolving solvents, antioxidants and their mixtures.

Suitable surface active agents may comprise but not limited to lecithin, menthol, polysorbate, diethanolamine, glyceryl monostearat, oleic acid, sodium lauril sulfate, polyethylene glycol succinate and mixtures thereof. Preferably the surface active agent is lecithin and the amount is from 0.10 to 20.0% lecithin, preferably from 1.0 to 15.0% by weight, more preferably from 2.0 to 10.0% by weight of the total composition, most preferably from 5.0 to 10.0% by weight of the total composition. These amounts of lecithin improve the spreading properties and minimize any balling up or drying of the gel compositions of the present invention when it is rubbed on the skin.

Suitable viscosity enhancers may comprise but not limited to sodium carboxyl methyl cellulose, glycerin, pullulan, dextran, cellulose and derivatives, chitosan, carbomer and mixtures thereof. Preferably the viscosity enhancer is sodium carboxyl methyl cellulose and the amount is from 0.050 to 10.0%, preferably from 0.50 to 5.0% by weight of the total composition, more preferably from 0.50 to 2.0% by weight of the total composition. Sodium carboxyl methyl cellulose has also a stabilisator effect when it is used in these amounts and helps the formulation to be stable over the shelf life.

Suitable chelating agents may comprise but not limited to sodium edetate, diethylenetriaminepentaacetic acid (DTPA) and N,N- bis(carboxymethyl) glycine (NTA).

Suitable aromatic agents may comprise but not limited to fenouil oil, eucalyptus oil, carnation oil, ginger oil, lavender oil, sweet orange oil, menthol and their mixtures.

Suitable preservatives may comprise but not limited to methylparaben, propylparaben, sodium and potassium benzoate, imidurea, monothioglicol, phenyl mercuric derivatives, sodium sulfate, sodium meta bisulfate, potassium sorbate or mixtures thereof. The amount of the preservatives are from 0.01 to 2.0% by weight of the total composition.

Suitable dissolving solvents may comprise but not limited to ethyl alcohol, polyethylene glycol, propylene glycol, isopropyl alcohol and purified water. Preferably ethyl alcohol, polyethylene glycol, propylene glycol and purified water are used. Ethyl alcohol is also utilized as a microbiological preservative.

Suitable antioxidants may comprise but not limited to alpha tocopherol, butyl hydroxy anisole, butyl hydroxyl toluene, monothioglicol, sodium meta bisulfate, potassium meta bisulfate and mixtures thereof. The amount of the antioxidants are from 0.0 to 2.0% by weight of the total composition.

The pharmaceutical compositions according to the present invention provides spreadable, semi-solid and jelly-like gel compositions of flurbiprofen or a pharmaceutically acceptable salt thereof with glucosamine or salts thereof and chondroitin sulfate. However, the topical compositions of the invention may also take the form of ointment, cream, spray or lotion. Accordingly, the present invention may be used for treating osteoarthritis, pain and inflammatory symptoms associated with joint and cartilage disorders.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example

| | | |
|---|---|---|
| **Content** | | **amount (%) (w/w)** |
| a. | flurbiprofen or pharmaceutically acceptable salts thereof | 5.0% by weight |
| b. | glucosamine sulfate | 5.0% by weight |
| c. | chondroitin sulfate | 1.0% by weight |
| d. | dimethyl sulfoxide | 10% by weight |
| e. | lecithin | 7.0% by weight |
| f. | fenouil oil | 1.0% by weight |
| g. | eucalyptus oil | 1.0% by weight |
| h. | carnation oil | 0.2% by weight |
| i. | ginger oil | 0.75% by weight |
| j. | lavender oil | 2.0% by weight |
| k. | sweet orange oil | 7.0% by weight |
| l. | menthol | 3.0% by weight |
| m. | methyl paraben | 0.30% by weight |
| n. | propyl paraben | 0.03% by weight |
| o. | disodium edetate | 0.10% by weight |
| p. | propylene glycol | 10.0% by weight |
| q. | polyethylene glycol | 10.0% by weight |
| r. | sodium carboxyl methyl cellulose | 1.0% by weight |
| s. | purified water | 10.0% by weight |
| t. | ethyl alcohol | about 30.0% by weight |

Flurbiprofen, methyl paraben, propyl paraben and menthol are added into ethyl alcohol until they dissolve. Then fenouil oil, eucalyptus oil, carnation oil, ginger oil, lavender oil, sweet orange oil, disodium edetate and lecithin are added and mixed 10 more minutes to obtain the mixture 1. Water is added to another container and chondroitin sulfate and glucosamine sulfate are mixed in this container until they dissolve. Then sodium carboxyl methyl cellulose, propylene glycol, polyethylene glycol and dimethyl sulfoxide are added into this container and mixed 30 more minutes to obtain mixture 2. The second mixture is added to the first mixture under stirring for about 10 min and filled up with ethyl alcohol. Then, it is brought into a gelled state and the procedure is completed and continue by filling step.

## Claims

1. A topical pharmaceutical gel composition comprising,
a. 0.50 to 30.0% by weight of flurbiprofen or pharmaceutically acceptable salts thereof,
b. 0.50 to 20.0% by weight of glucosamine or salts thereof,
c. 0.10 to 20.0% by weight of chondroitin sulfate,
d. 0.10 to 30.0% by weight of dimethyl sulfoxide.

2. The topical pharmaceutical gel composition according to claim 1, wherein the amount of dimethyl sulfoxide is preferably from 0.50 to 25.0% by weight of the total composition, more preferably it is from 1.0 to 20.0% by weight of the total composition, most preferably it is from 5.0 to 15.0% by weight of the total composition.

3. The topical pharmaceutical gel composition according to claim 1, wherein glucosamine salt is glucosamine sulfate.

4. The topical pharmaceutical gel composition according to claims 1 to 3, further comprising lecithin in the amount of from 0.10 to 20.0% by weight of the total composition.

5. The topical pharmaceutical gel composition according to claims 1 to 4, further comprising sodium carboxyl methyl cellulose in the amount of from 0.05 to 10.0% by weight of the total composition.

6. The topical pharmaceutical gel composition according to claims 4 and 5, wherein the weight ratio of lecithin to sodium carboxyl methyl cellulose is from 25:1 to 1:25 by weight, preferably it is from 10:1 to 1:10 by weight of the total composition.

7. The topical pharmaceutical gel composition according to any of the preceding claims, further comprising menthol.

8. The topical pharmaceutical gel composition according to claim 7, wherein the amount of menthol is between 0.10 to 15.0 % by weight of the total composition, preferably it is 0.50 to 10.0 % by weight of the total composition.

9. The topical pharmaceutical gel composition according to any of the preceding claims, further comprising surface active agents, viscosity enhancers, chelating agents, aromatic agents, preservatives, dissolving solvents, antioxidants and their mixtures.

10. The topical pharmaceutical gel composition according to claim 9, wherein the chelating agent is disodium edetate, in the amount of 0.005 to 5.0 % by weight of the total composition, preferably it is 0.05 to 2.0 % by weight of the total composition.

11. The topical pharmaceutical gel composition according to any of the preceding
claims, comprising,
| | | |
|---|---|---|
| a. | flurbiprofen or pharmaceutically acceptable salts thereof | 0.50 to 30.0% by weight |
| b. | glucosamine sulfate | 0.50 to 20.0% by weight |
| c. | chondroitin sulfate | 0.10 to 20.0% by weight |
| d. | dimethyl sulfoxide | 0.10 to 30.0% by weight |
| e. | lecithin | 0.10 to 20.0% by weight |
| f. | fenouil oil | 0.01 to 15.0% by weight |
| g. | eucalyptus oil | 0.01 to 15.0% by weight |
| h. | carnation oil | 0.01 to 15.0% by weight |
| i. | ginger oil | 0.01 to 15.0% by weight |
| j. | lavender oil | 0.01 to 15.0% by weight |
| k. | sweet orange oil | 0.10 to 15.0% by weight |
| l. | menthol | 0.10 to 15.0% by weight |
| m. | methyl paraben | 0.01 to 2.0% by weight |
| n. | propyl paraben | 0.01 to 2.0% by weight |
| o. | disodium edetate | 0.005 to 5.0% by weight |
| p. | propylene glycol | 1.0 to 30.0% by weight |
| q. | polyethylene glycol | 1.0 to 50.0% by weight |
| r. | sodium carboxyl methyl cellulose | 0.05 to 10.0% by weight |
| s. | purified water | 1.0 to 50.0% by weight |
| t. | ethyl alcohol | 1.0 to 75.0% by weight |

12. Method for preparing the topical pharmaceutical gel composition according to any of the preceding claims, comprising the steps of;
a. mixing flurbiprofen, methyl paraben, propyl paraben and menthol in ethyl alcohol until they dissolve. Then adding fenouil oil, eucalyptus oil, carnation oil, ginger oil, lavender oil, sweet orange oil, disodium edetate and lecithin and mixing them 10 more minutes to obtain the mixture 1.
b. adding water to another container and mixing chondroitin sulfate and glucosamine sulfate in this container until they dissolve. Then adding sodium carboxyl methyl cellulose, propylene glycol, polyethylene glycol and dimethyl sulfoxide and mixing them 30 more minutes to obtain mixture 2.
c. adding the second mixture into the first mixture and stirring 10 more minutes.

13. The topical pharmaceutical gel composition according to any previous claims, for use in the treatment of osteoarthritis, pain and inflammatory symptoms associated with joint and cartilage disorders.

## Patentansprüche

1. Topische pharmazeutische Gelzusammensetzung umfassend,
a. 0,50 bis 30,0 Gewichtsprozent Flurbiprofen oder pharmazeutisch verträgliche Salze davon,
b. 0,50 bis 20,0 Gewichtsprozent Glucosamin oder Salze davon,
c. 0,10 bis 20,0 Gewichtsprozent Chondroitinsulfat,
d. 0,10 bis 30,0 Gewichtsprozent Dimethylsulfoxid.

2. Topische pharmazeutische Gelzusammensetzung nach Anspruch 1, wobei die Menge an Dimethylsulfoxid bevorzugt von 0,50 bis 25,0 Gewichtsprozent der gesamten Zusammensetzung beträgt, stärker bevorzugt von 1,0 bis 20,0 Gewichtsprozent der gesamten Zusammensetzung beträgt, am stärksten bevorzugt von 5,0 bis 15,0 Gewichtsprozent der gesamten Zusammensetzung beträgt.

3. Topische pharmazeutische Gelzusammensetzung nach Anspruch 1, wobei das Glucosaminsalz Glucosaminsulfat ist.

4. Topische pharmazeutische Gelzusammensetzung nach Ansprüchen 1 bis 3, des Weiteren umfassend Lecithin in der Menge von 0,10 bis 20,0 Gewichtsprozent der gesamten Zusammensetzung.

5. Topische pharmazeutische Gelzusammensetzung nach Ansprüchen 1 bis 4, des Weiteren umfassend Natriumcarboxylmethylzellulose in der Menge von 0,05 bis 10,0 Gewichtsprozent der gesamten Zusammensetzung.

6. Topische pharmazeutische Gelzusammensetzung nach Ansprüchen 4 und 5, wobei das Gewichtsverhältnis von Lecithin zu Natriumcarboxylmethylcellulose von 25:1 bis 1:25 bezogen auf das Gewicht beträgt, vorzugsweise von 10:1 bis 1:10 bezogen auf das Gewicht der gesamten Zusammensetzung beträgt.

7. Topische pharmazeutische Gelzusammensetzung nach einem beliebigen der vorhergehenden Ansprüche, des Weiteren umfassend Menthol.

8. Topische pharmazeutische Gelzusammensetzung nach Anspruch 7, wobei die Menge an Menthol zwischen 0,10 bis 15,0 Gewichtsprozent der gesamten Zusammensetzung liegt, vorzugsweise 0,50 bis 10,0 Gewichtsprozent der gesamten Zusammensetzung beträgt.

9. Topische pharmazeutische Gelzusammensetzung nach einem beliebigen der vorhergehenden Ansprüche, des Weiteren umfassend oberflächenaktive Mittel, Viskositätsverstärker, Chelatbildner, aromatische Mittel, Konservierungsstoffe, auflösende Lösemittel, Antioxidantien und deren Mischungen.

10. Topische pharmazeutische Gelzusammensetzung nach Anspruch 9, wobei der Chelatbildner Dinatriumedetat ist, in der Menge von 0,005 bis 5,0 Gewichtsprozent der gesamten Zusammensetzung, bevorzugt 0,05 bis 2,0 Gewichtsprozent der gesamten Zusammensetzung beträgt.

11. Topische pharmazeutische Gelzusammensetzung nach Ansprüchen nach einem beliebigen der vorhergehenden Ansprüche, umfassend,
| | | |
|---|---|---|
| a. | Flurbiprofen oder pharmazeutisch verträgliche Salze davon | 0,50 bis 30,0 Gewichtsprozent |
| b. | Glucosaminsulfat | 0,50 bis 20,0 Gewichtsprozent |
| c. | Chondroitinsulfat | 0,10 bis 20,0 Gewichtsprozent |
| d. | Dimethylsulfoxid | 0,10 bis 30,0 Gewichtsprozent |
| e. | Lecithin | 0,10 bis 20,0 Gewichtsprozent |
| f. | Fenchelöl | 0,01 bis 15,0 Gewichtsprozent |
| g. | Eukalyptusöl | 0,01 bis 15,0 Gewichtsprozent |
| h. | Nelkenöl | 0,01 bis 15,0 Gewichtsprozent |
| i. | Ingweröl | 0,01 bis 15,0 Gewichtsprozent |
| j. | Lavendelöl | 0,01 bis 15,0 Gewichtsprozent |
| k. | Süßorangenöl | 0,10 bis 15,0 Gewichtsprozent |
| l. | Menthol | 0,10 bis 15,0 Gewichtsprozent |
| m. | Methylparaben | 0,01 bis 2,0 Gewichtsprozent |
| n. | Propylparaben | 0,01 bis 2,0 Gewichtsprozent |
| o. | Dinatriumedetat | 0,005 bis 5,0 Gewichtsprozent |
| p. | Propylenglycol | 1,0 bis 30,0 Gewichtsprozent |
| q. | Polyethylenglycol | 1,0 bis 50,0 Gewichtsprozent |
| r. | Natriumcarboxylmethylcellulose | 0,05 bis 10,0 Gewichtsprozent |
| s. | gereinigtes Wasser | 1,0 bis 50,0 Gewichtsprozent |
| t. | Ethylalkohol | 1,0 bis 75,0 Gewichtsprozent |

12. Verfahren zur Herstellung der topisch pharmazeutischen Gelzusammensetzung nach einem beliebigen der vorhergehenden Ansprüche umfassend die Schritte von;
a. Mischen von Flurbiprofen, Methylparaben, Propylparaben und Menthol in Ethylalkohol bis sie sich lösen; dann Hinzufügen von Fenchelöl, Eukalyptusöl, Nelkenöl, Ingweröl, Lavendelöl, Süßorangenöl, Dinatriumedetat und Lecithin und Mischen davon für weitere 10 Minuten um die Mischung 1 zu erhalten;
b. Hinzufügen von Wasser in einen anderen Behälter und Mischen von Chondroitinsulfat und Glucosaminsulfat in diesem Behälter bis sie sich lösen; dann Hinzufügen von Natriumcarboxylmethylcellulose, Propylenglycol, Polyethylenglycol und Dimethylsulfoxid und Mischen davon für weitere 30 Minuten um die Mischung 2 zu erhalten;
c. Hinzufügen der zweiten Mischung in die erste Mischung und Rühren für 10 weitere Minuten.

13. Topische pharmazeutische Gelzusammensetzung nach einem beliebigen der vorhergehenden Ansprüche, zur Verwendung in der Behandlung von Osteoarthritis, Schmerzen und Entzündungserscheinungen die mit Gelenk- und Knorpelerkrankungen assoziiert sind.

## Revendications

1. Composition de gel pharmaceutique topique comprenant,
a. 0,50 à 30,0 % en poids de flurbiprofène ou de sels pharmaceutiquement acceptables de celui-ci,
b. 0,50 à 20,0 % en poids de glucosamine ou de sels de celle-ci,
c. 0,10 à 20,0 % en poids de sulfate de chondroïtine,
d. 0,10 à 30,0 % en poids de sulfoxyde de diméthyle.

2. Composition de gel pharmaceutique topique selon la revendication 1, dans laquelle la quantité de sulfoxyde de diméthyle est de préférence de 0,50 à 25,0 % en poids de la composition totale, de manière davantage préférée elle est de 1,0 à 20,0 % en poids de la composition totale, de manière préférée entre toutes elle est de 5,0 à 15,0 % en poids de la composition totale.

3. Composition de gel pharmaceutique topique selon la revendication 1, dans laquelle le sel de glucosamine est le sulfate de glucosamine.

4. Composition de gel pharmaceutique topique selon les revendications 1 à 3, comprenant en outre de la lécithine dans la quantité de 0,10 à 20,0 % en poids de la composition totale.

5. Composition de gel pharmaceutique topique selon les revendications 1 à 4, comprenant en outre de la carboxyméthylcellulose sodique dans la quantité de 0,05 à 10,0 % en poids de la composition totale.

6. Composition de gel pharmaceutique topique selon les revendications 4 et 5, dans laquelle le rapport pondéral de la lécithine sur la carboxyméthylcellulose sodique est de 25/1 à 1/25 en poids, de préférence il est de 10/1 à 1/10 en poids de la composition totale.

7. Composition de gel pharmaceutique topique selon l'une quelconque des revendications précédentes, comprenant en outre du menthol.

8. Composition de gel pharmaceutique topique selon la revendication 7, dans laquelle la quantité de menthol se situe entre 0,10 et 15,0 % en poids de la composition totale, de préférence elle est de 0,50 à 10,0 % en poids de la composition totale.

9. Composition de gel pharmaceutique topique selon l'une quelconque des revendications précédentes, comprenant en outre des agents tensioactifs, des amplificateurs de la viscosité, des agents chélateurs, des agents aromatiques, des conservateurs, des agents de dissolution, des antioxydants et leurs mélanges.

10. Composition de gel pharmaceutique topique selon la revendication 9, dans laquelle l'agent chélateur est l'édétate disodique, dans la quantité de 0,005 à 5,0 % en poids de la composition totale, de préférence elle est de 0,05 à 2,0 % en poids de la composition totale.

11. Composition de gel pharmaceutique topique selon l'une quelconque des
revendications précédentes, comprenant,
| | | |
|---|---|---|
| a. | flurbiprofène ou sels pharmaceutiquement acceptables de celui-ci | 0,50 à 30,0 % en poids |
| b. | sulfate de glucosamine | 0,50 à 20,0 % en poids |
| c. | sulfate de chondroïtine | 0,10 à 20,0 % en poids |
| d. | sulfoxyde de diméthyle | 0,10 à 30,0 % en poids |
| e. | lécithine | 0,10 à 20,0 % en poids |
| f. | huile de fenouil | 0,01 à 15,0 % en poids |
| g. | huile d'eucalyptus | 0,01 à 15,0 % en poids |
| h. | huile d'oeillet | 0,01 à 15,0 % en poids |
| i. | huile de gingembre | 0,01 à 15,0 % en poids |
| j. | huile de lavande | 0,01 à 15,0 % en poids |
| k. | huile d'orange douce | 0,10 à 15,0 % en poids |
| l. | menthol | 0,10 à 15,0 % en poids |
| m. | parabenzoate de méthyle | 0,01 à 2,0 % en poids |
| n. | parabenzoate de propyle | 0,01 à 2,0 % en poids |
| o. | édétate disodique | 0,005 à 5,0 % en poids |
| p. | propylène glycol | 1,0 à 30,0 % en poids |
| q. | polyéthylène glycol | 1,0 à 50,0 % en poids |
| r. | carboxyméthylcellulose sodique | 0,05 à 10,0 % en poids |
| s. | eau purifiée | 1,0 à 50,0 % en poids |
| t. | alcool éthylique | 1,0 à 75,0 % en poids |

12. Procédé de préparation de la composition de gel pharmaceutique topique selon l'une quelconque des revendications précédentes, comprenant les étapes suivantes :
a. le mélange du flurbiprofène, du parabenzoate de méthyle, du parabenzoate de propyle et du menthol dans l'alcool éthylique jusqu'à leur dissolution. Puis l'addition de l'huile de fenouil, de l'huile d'eucalyptus, de l'huile d'oeillet, de l'huile de gingembre, de l'huile de lavande, de l'huile d'orange douce, de l'édétate disodique et de la lécithine et leur mélange pendant plus de 10 minutes pour obtenir le mélange 1 ;
b. l'addition d'eau dans un autre récipient et le mélange du sulfate de chondroïtine et du sulfate de glucosamine dans ce récipient jusqu'à leur dissolution. Puis l'addition de la carboxyméthylcellulose sodique, du propylène glycol, du polyéthylène glycol et du sulfoxyde de diméthyle et leur mélange pendant plus de 30 minutes pour obtenir le mélange 2 ;
c. l'addition du second mélange dans le premier mélange et l'agitation pendant plus de 10 minutes.

13. Composition de gel pharmaceutique topique selon l'une quelconque des revendications précédentes, pour une utilisation dans le traitement de l'arthrose, de la douleur et des symptômes inflammatoires associés à des troubles articulaires et cartilagineux.
